# EUROPEAN PATENT SPECIFICATION

(11) **EP 0 481 533 B1**
(45) Date of publication and mention of the grant of the patent: **05.03.1997**
(21) Application number: 91121541.6
(22) Date of filing: 17.07.1985
(51) Int. Cl.: C08K 5/3435, C08K 5/13, C08K 5/36, C08K 5/52

(54) **Stabilized resin composition**
Stabilisierte Harzzusammensetzung
Composition de résine stabilisée

(30) Priority: 24.07.1984 JP 154633/84
(43) Date of publication of application: 22.04.1992
(62) Divisional of application: 85108956.5
(73) Proprietor: SUMITOMO CHEMICAL COMPANY LIMITED, Osaka-shi, Osaka 541 (JP)
(72) Inventor: Fujii, Takeo, Toyonaka-shi (JP); Ishii, Tamaki, Suita-shi (JP); Yachigo, Shinichi, Toyonaka-shi (JP); Kaneoya, Tatsuo, Toyonaka-shi (JP); Takahashi, Yukoh, Toyonaka-shi (JP); Maegawa, Yuzo, Ibaraki-shi (JP); Okamura, Haruki, Joto-ku, Osaka (JP); Okino, Eizo, Nishinomiya-shi (JP)
(74) Representative: Henkel, Feiler, Hänzel & Partner

(56) References cited:
- EP-A- 0 062 322
- DE-A- 3 233 353

## Description

### BACKGROUND OF THE INVENTION

### FIELD OF THE INVENTION

This invention relates to a stabilized resin composition containing a piperidine derivative represented by the general formula (III) 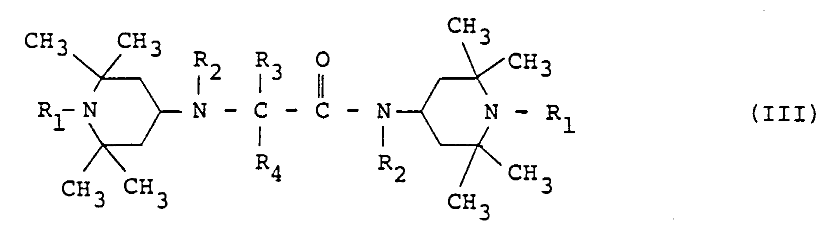 wherein R₁ is a hydrogen atom or an alkyl group having 1 to 3 carbon atoms; R₂ is a hydrogen atom or an alkyl group having 1 to 12 carbon atoms; and R₃ and R₄ is each independently an alkyl group having 1 to 12 carbon atoms.

The resin composition containing said piperidine derivative which has extremely excellent stability not only to heat and oxidation but also to light.

### DESCRIPTION OF THE PRIOR ART

The piperidine derivative represented by the above-mentioned general formula (III) is known to be useful as a light stabilizer for high molecular substances such as plastics, rubbers and fibers (see for example EP-A-62322).

It is well known that such resins as polyethylene, polypropylene, polyvinyl chloride, polyurethane and ABS resin deteriorate by the action of heat, light and oxygen and undergo marked decrease of mechanical properties accompanied by such phenomena as softening, brittleness, surface crazing and discoloration.

It has been already known for the purpose of preventing such deterioration caused by heat and oxidation to use various kinds of phenol-type compounds such as 2,6-di-t-butyl-4-methylphenol and tris(3,5-di-t-butyl-4-hydroxybenzyl) isocyanurate; for the purpose of further improving the oxidation preventing property to use sulfur-containing compounds such as dilauryl thiodipropionate and pentaerythritol tetrakis-(3-dodecylthiopropionate) in combination with said phenol-type antioxidant; and further for the purpose of preventing deterioration by light to use jointly light stabilizers including, for example, benzophenone compounds such as 2-hydroxy-4-n-octoxy-benzophenone, benzotriazole compounds such as 2-(2-hydroxy-3-t-butyl-5-methylphenyl)-5-chloro-benzotriazole and 2-(2-hydroxy-3,5-dipentylphenyl)benzotriazole, cyanoacrylate compounds such as 2-cyano-3,3-diphenylacrylate, Ni-containing compounds such as Ni salt of bis(3,5-di-t-butyl-4-hydroxybenzylphosphoric acid) monoethyl ester, and hindered piperidine compounds such as 4-benzoyloxy-2,2,6,6-tetramethylpiperidine, bis(2,2,6,6-tetramethyl-4-piperidyl) sebacate, and a reaction product of N,N'-bis(2,2,6,6-tetramethyl-4-piperidyl)hexylenediamine with 2,4-dichloro-6-t-octylamino-1,3,5-triazine.

However, even in stabilized resin compositions obtained by using these known stabilizers in combination, the requirements for heat resistance, oxidation resistance and light resistance are not met simultaneously.

For example, when a phenol-type compound and a sulfur-containing compound are used as antioxidants, and a benzophenone compound, benzotirazole compound, cyanoacrylate compound, Ni-containing compounds, or the like is used jointly as a light stabilizer, the resulting products are not fully satisfactory in their light resistance. Further, in cases where hindered piperidine compounds commonly used as a light stabilizer are employed, when a sulfur-containing stabilizer is used in combination therewith, there occurs, presumably owing to antagonism, a serious problem that the excellent light-stabilizing effect inherent to the hindered piperidine compounds is extremely lowered. Consequently, selections have to be made between either to use jointly phenol-type stabilizers alone without using sulfur-containing stabilizer to enhance the light resistance at the sacrifice of the resistance to heat and oxidation or alternatively to use also sulfur-containing stabilizers together to enhance the resistance to heat and oxidation at the sacrifice of the light resistance. Thus, even stabilizer systems using a hindered piperidine compound in combination cannot give fully satisfactory resistances to heat, oxidation and light simultaneously.

The present inventors have made intensive studies to solve these problems and resultantly found that the requirements for heat resistance, oxidation resistance and light resistance can be satisfied simultaneously by using a specified piperidine derivative in combination with a sulfur-containing antioxidant and a phenol-type antioxidant without extreme lowering of light resistance as experienced in combinations of various conventional hindered piperidine compounds with sulfur-containing antioxidants, and thus accomplished this invention.

### SUMMARY OF THE INVENTION

An object of this invention is to provide a stabilized resin composition showing high heat, oxidation and light stability.

According to this invention, there is provided an extremely excellent stabilized resin composition comprising a resin, a piperidine derivative represented by the general formula (III) 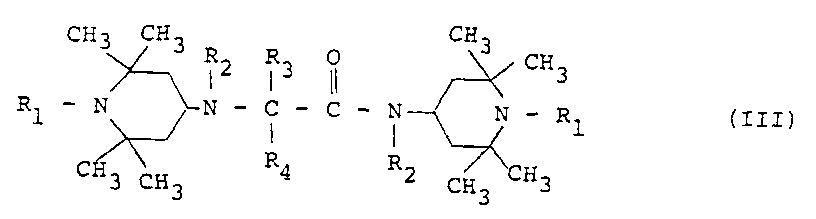 wherein R₁ is a hydrogen atom or an alkyl group having 1 to 3 carbon atoms; R₂ is a hydrogen atom or an alkyl group having 1 to 12 carbon atoms; and R₃ and R₄ are each independently an alkyl group having 1 to 12 carbon atoms, a phenol-type antioxidant and a sulfur-containing antioxidant which composition can satisfy the requirements not only for resistance to heat and oxidation but also simultaneously resistance to light.

The piperidine derivatives of the general formula (III) are obtained by reacting a piperidine represented by the general formula (I) 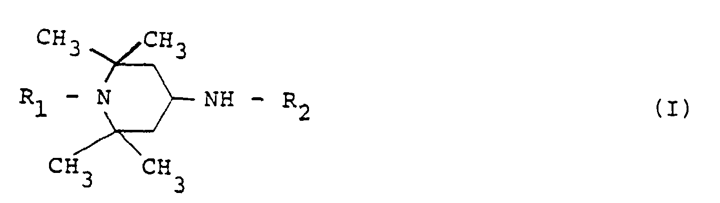 wherein R₁ and R₂ are as defined above,
with a ketone represented by the general formula (II) 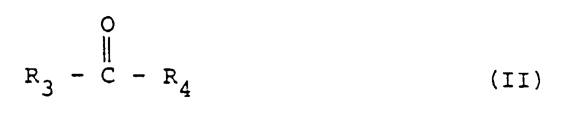 wherein R₃ and R₄ are as defined above,
in the presence of chloroform and alkali and in the presence or absence of a phase transfer catalyst or according known processes.

Details concerning the reactants, phase transfer catalysts and reaction conditions are disclosed in EP-A-0172413.

Typical piperidine derivatives of the general formula (III) to be used in the stabilized resin composition of this invention are shown in Table 1.

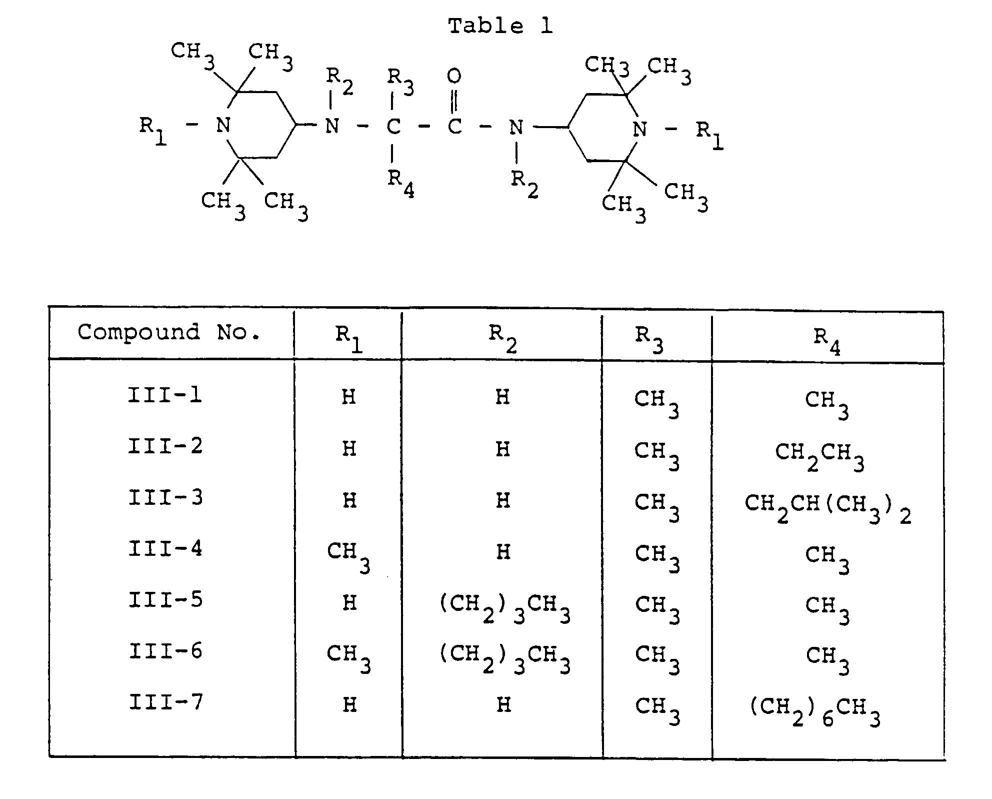

In obtaining the stabilized resin composition of this invention, from the viewpoint of obtaining more excellent light stability, the substituent R₁ in the above-mentioned general formula (III) is preferably a hydrogen atom or a methyl group, more preferably a hydrogen atom; R₂ is preferably a hydrogen atom or an alkyl group having 1 to 4 carbon atoms, more preferably a hydrogen atom; R₃ and R₄ are, each independently, preferably an alkyl group of 1 to 7 carbon atoms, more preferably a methyl group.

Examples of phenol-type antioxidants used for obtaining the stabilized resin composition of this invention are selected from the group consisting of 2,6-di-t-butyl-4-methylphenol, n-octadecyl 3-(3,5-di-t-butyl-4-hydroxyphenyl)propionate, 2,2'-methylenebis(4-methyl-6-t-butylphenol), 4,4'-butylidenebis(3-methyl-6-t-butylphenol), 4,4'-thiobis(3-methyl-6-t-butylphenol), 2-t-butyl-6-(3-t-butyl-5-methyl-2-hydroxybenzyl)-4-methylphenyl acrylate, 1,1,3-tris(2-methyl-4-hydroxy-5-t-butylphenyl)butane, 1,3,5-trimethyl-2,4,6-tris(3-alkyl 5-t-butyl-4-hydroxybenzyl)benzene, 1,3,5-tris (3-alkyl-5-t-butyl-4-hydroxybenzyl)isocyanurate, 1,3,5-tris(3-(3-alkyl-5-t-butyl-4-hydroxyphenyl)propionyloxyethyl) isocyanurate, ethylene glycol bis(3,3-bis(4-hydroxy-3-t-butylphenyl )butanoate, and pentaerythritol tetrakis(3-(3-alkyl-5-t-butyl-4-hydroxyphenyl)propionate).

Examples of sulfur-containing antioxidants used in this invention are selected from the group consisting of dilauryl thiodipropionate, dimyristyl thiodipropionate, distearyl thiodipropionate, pentaerythritol tetrakis(3-dodecylthiopropionate), and 3,9-bis(2-dodecylthioethyl)-2,4,8,10-tetraoxaspiro [5.5] undecane.

The stabilized resin composition of this invention contains a specified piperidine derivative represented by the above-mentioned general formula (III), a phenol-type antioxidant and a sulfer-containing antioxidant. The total content of these stabilizers (i.e. of the piperidine derivative, the phenol-type antioxidant and the sulfur-containing antioxidant) is usually 0.01 to 5 parts by weight, preferably 0.05 to 2 parts by weight, based on 100 parts by weight of resin. The weight ratio of respective stabilizers is normally 1-20 : 1 : 1-15 in terms of compound (III) : phenol-type antioxidant : sulfur-containing antioxidant.

For compounding the resin composition, conventional apparatuses and operation procedures used for incorporating stabilizers, pigments, fillers and the like into resins can be employed with little or no alteration.

The stabilizers for resin of this invention may be used jointly with other ingredients including, for example, antioxidants, light stabilizers, sequestering agents, metallic soaps, nucleating agents, lubricants, antistatic agents, flame retardants, pigments and fillers.

Particularly, the color of the resin composition can be improved by jointly using phosphite antioxidants as the antioxidants.

Examples of the phosphite antioxidants include tris(nonylphenyl) phosphite, distearyl pentaerythritol diphosphite, tris(2,4-di-t-butylphenyl) phosphite, tris(2-t-butyl-4-methylphenyl) phosphite, bis(2,4-di-t-butylphenyl) pentaerythritol diphosphite, and tetrakis(2,4-di-t-butylphenyl)-4,4'-biphenylene diphosphonite.

The light stability of the composition of this invention can be further improved by adding light stabilizers other than the above-mentioned piperidine derivatives represented by the general formula (III). These light stabilizers include, for example, benzophenone compounds such as 2-hydroxy-4-methoxybenzophenone, 2-hydroxy-n-octoxybenzophenone and 2,2'-dihydroxy-4,4'-dimethoxybenzophenone; benzotriazole compounds such as 2-(2-hydroxy-3-t-butyl-5-methylphenyl)-5-chlorobenzotriazole, 2-(2-hydroxy-3,5-dipentylphenyl)benzotriazole, 2-(2-hydroxy-3-t-butyl-5-methylphenyl)benzotriazole, 2-(2-hydroxy-5-methylphenyl)benzotriazole, 2-(2-hydroxy-3,5-di-t-butylphenyl)benzotriazole, 2-(2-hydroxy-3,5-di-t-butylphenyl)-5-chlorobenzotriazole, 2-(2-hydroxy-5-t-octylphenyl)benzotriazole, and 2-(2-hydroxy-3,5-di-(α,α-dimethylbenzyl)phenyl)benzotriazole; benzoate-type compounds such as phenyl salicylate, p-t-butylphenyl salicylate, 2,4-di-t-butylphenyl 3',5'-di-t-butyl-4'-hydroxybenzoate, and hexadecyl 3,5-di-t-butyl-4-hydroxybenzoate: Ni compounds such as Ni salt of dibutyldithiocarbamic acid, [2,2'-thiobis(4-t-octylphenolate)]-n-butylamine Ni complex and Ni salt of bis(3,5-di-t-butyl-4-hydroxybenzylphosphoric acid) monoethyl ester; cyanoacrylate compounds such as ethyl 2-cyano-3,3-diphenylacrylate; and oxalic acid diamides such as N-2-ethylphenyl-N'-2-ethoxy-5-t-butylphenyl oxalic acid diamide and N-2-ethylphenyl-N'-2-ethoxyphenyl oxalic acid diamide.

Examples of resins which can be stabilized according to this invention include poly-α-olefins such as low density polyethylene, medium to high density polyethylene, linear low density polyethylene, polypropylene and polybutene-1; α-olefin copolymers such as propylene-ethylene random or block copolymer and ethylene-butene-1 random copolymer; copolymers of poly-α-olefin with vinyl monomers such as maleic anhydride-modified polypropylene; or mixtures thereof; chlorinated polyethylene, EVA resin, polyvinyl chloride, methacrylic resin, polystyrene, high impact polystyrene, ABS resin, AES resin, MBS resin, polyethylene terephthalate, polybutylene terephthalate, polyamide, polyimide, polycarbonate, polyacetal, polyurethane, and unsaturated polyester resin. Further, these resins may be blended with rubbers such as isoprene rubber, butadiene rubber, acrylonitrile-butadiene copolymer rubber, styrene-butadiene copolymer rubber and ethylene-propylene rubber.

This invention will be described in more detail below with reference to the Reference Example and to the Examples.

### Reference Example (Preparation of representative piperidine derivatives of general formula (III))

### A) Preparation of compound No. III-1

Into a four-necked flask equipped with a thermometer and a stirrer, were placed 156 g of 4-amino-2,2,6,6-tetramethylpiperidine, 32 g of acetone, 239 g of chloroform and 0.4 g of benzyltrimethylammonium chloride, and the mixture was cooled down to 5°C with stirring. Then, 257.6 g of 50% aqueous potassium hydroxide solution was added dropwise thereto over a period of 1 hour while the inner temperature was maintained at 5 to 10°C, and the resulting mixture was allowed to react at a temperature of 5 to 10°C for 5 hours.

After completion of the reaction, the reaction liquid was separated into two layers, the aqueous layer was discarded, and excess chloroform in the organic layer was distilled off to obtain 176 g of white crystals. Yield: 92.6%; m.p.: 127-128°C. Parent ion peak of 380 was confirmed by FD-mass analysis.

### B) Preparation of compound No. III-2

Reaction and after-treatment were carried out in the same manner as in A) above except that 39.6 g of methyl ethyl ketone was used in place of acetone used in the Example to obtain 177 g of white crystals. Yield: 89.8%; m.p.: 108-109°C. Parent ion peak of 394 was confirmed by FD-mass analysis.

### C) Preparation of compound No. III-3

Reaction was carried out in the same manner as in A) above except that 55 g of methyl isobutyl ketone was used in place of acetone used in the Example. After removing excess chloroform by distillation and recrystalized from n-hexane, 175 g of white crystals were obtained. Yield: 80.8%; m.p.: 109-110°C. Parent ion peak of 422 was confirmed by FD-mass analysis.

### D) Preparation of compound No. III-4

Reaction and after-treatment were carried out in the same manner as in A) above except that 170 g of 4-amino-1,2,2,6,6-pentamethylpiperidine was used in place of the piperidine compound used in the Example to obtain 184 g of a viscous liquid in 90.2% yield. Parent ion peak of 408 was confirmed by FD-mass analysis.

### E) Preparation of compound No. III-7

Reaction and after-treatment were carried out in the same manner as in C) above except that 78 g of 2-nonanone was used in place of methyl isobutyl ketone used in the Example to obtain 168 g of white crystals. Yield: 72.4%; m.p.: 100-101°C. Parent ion peak of 464 was confirmed by FD-mass analysis.

### F) Preparation of compound No. III-1

Into a four-necked flask equipped with a thermometer and a stirrer, were placed 100 g of 4-amino-2,2,6,6-tetramethylpiperidine, 600 g of acetone and 54.5 g of chloroform, and the mixture was cooled down to 5°C with stirring. Then, 64 g of a solid potassium hydroxide was added little by little over a period of one hour, while the inner temperature was maintained at 5 to 10°C, and the resulting mixture was allowed to react at a temperature of 0 to 10°C for 4 hours.

After completion of the reaction, potassium chloride formed was separated by filtration, and 500 g of acetone was distilled off from the filtrate (acetone solution). White crystals isolated from the concentrated acetone solution were filtered and dried to obtain 110 g of the product. Yield: 91%; m.p.: 127-128°C. Parent ion peak of 380 was confirmed by FD-mass analysis.

### G) Preparation of compound No. III-2

Reaction and after-treatment were carried out in the same manner as in F) except that methyl ethyl ketone was used in place of acetone to obtain 108 g of white crystals. Yield: 85.3%; m.p.: 108-109°C. Parent ion peak of 394 was confirmed by FD-mass analysis.

### H) Preparation of compound No. III-3

Reaction and after-treatment were carried out in the same manner as in F) except that methyl isobutyl ketone was used in place of acetone and the resulting product was recrystalized from n-hexane to obtain 107 g of white crystals. Yield: 76.3%; m.p.: 109-110°C. Parent ion peak of 422 was confirmed by FD-mass analysis.

### (I) Preparation of compound No. III-4

Reaction and after-treatment were carried out in the same manner as in F) above except that 109 g of 4-amino-1,2,2,6,6-pentamethylpiperidine was used in place of 100 g of 4-amino-2,2,6,6-tetramethylpiperidine to obtain 118 g of a viscous liquid. Yield: 90.2%. Parent ion peak of 408 was confirmed by FD-mass analysis.

### J) Preparation of compound No. III-7

Reaction and after-treatment were carried out in the same manner as in H) above except that 2-nonane was used in place of methyl isobutyl ketone to obtain 116 g of white crystals. Yield: 78.2%; m.p.: 100-101°C. Parent ion peak of 464 was confirmed by FD-mass analysis.

### Examples 1 to 13 and Comparative Examples I to XXXI

Compounding ingredients shown below were blended in a mixer for 5 minutes and then melt-kneaded in mixing rolls at 180°C. The compound thus obtained was formed into a sheet of 1 mm thickness in a hot press at 210°C, from which test pieces 150 x 30 x 1 mm in size were prepared.

The test pieces were irradiated with light in a Sunshine Weather-O-meter (light source: carbon arc, black panel temperature: 83 ± 3°C, spraying cycle: 120 minutes, spraying time: 18 minutes) and were bent almost double every 60 hours. The time which had elapsed until the specimen broke by bending was determined to evaluate the weather resistance of the specimen.

Meanwhile, test pieces 40 x 40 x 1 mm in size were prepared and placed in a Geer oven at 160°C to determine the period of time which had elapsed until 30% of the one side surface of the test piece became brittle. The time was regarded as the induction period for thermal brittleness and used to evaluate thermal and oxidation stability.

The results obtained are shown in Table 2.

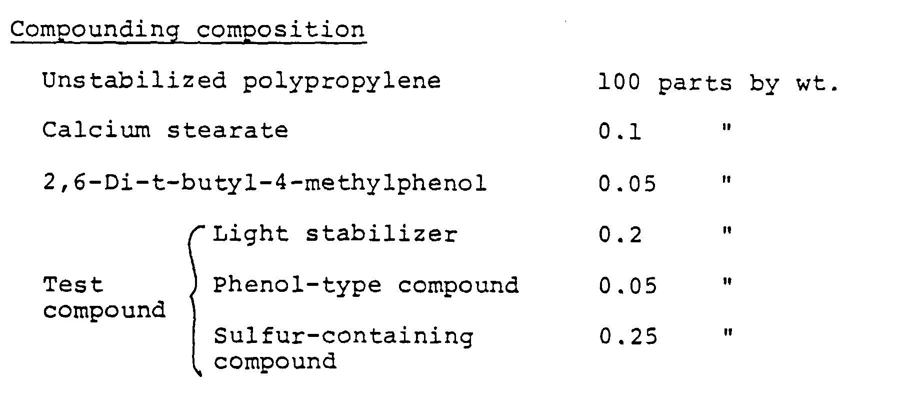

In Table 2, UVA-1 to AO-3 refer to the following compounds.

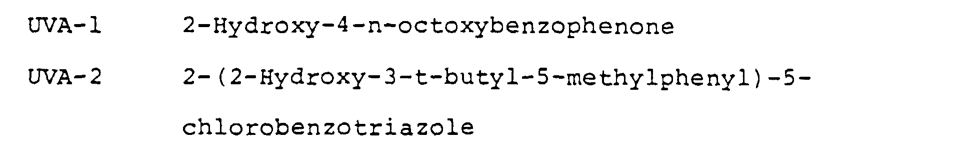

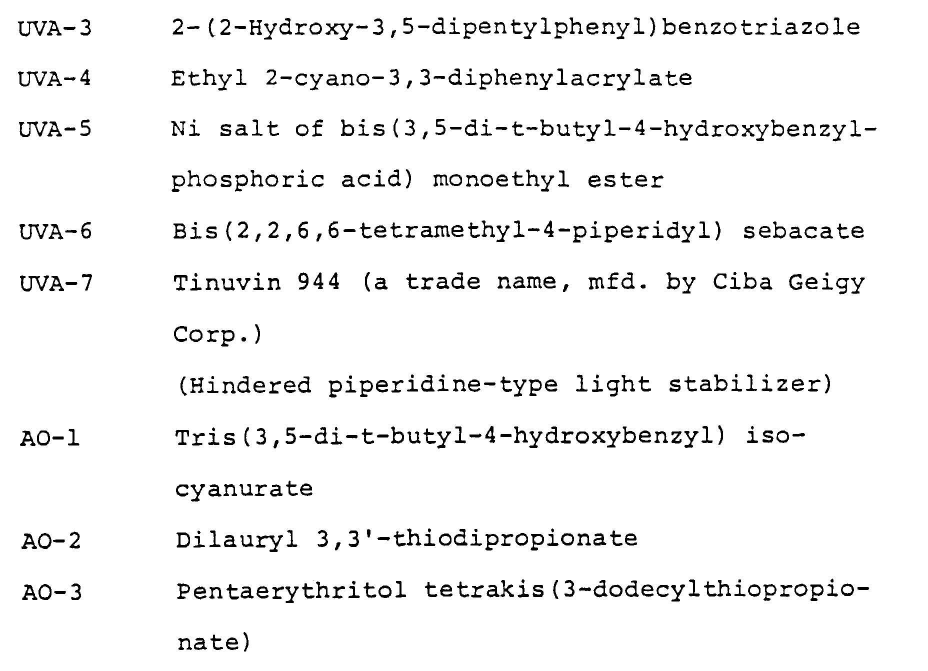

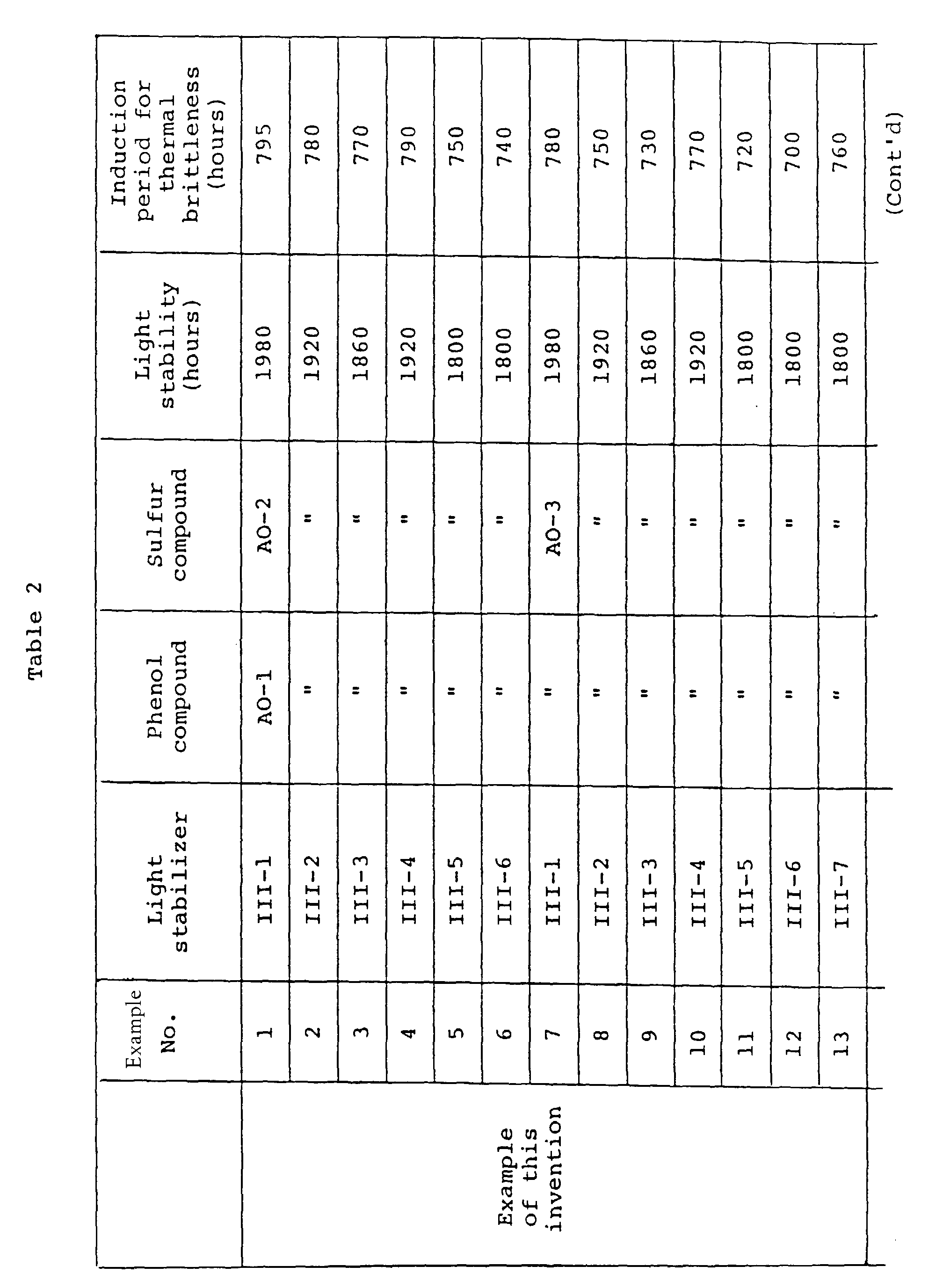

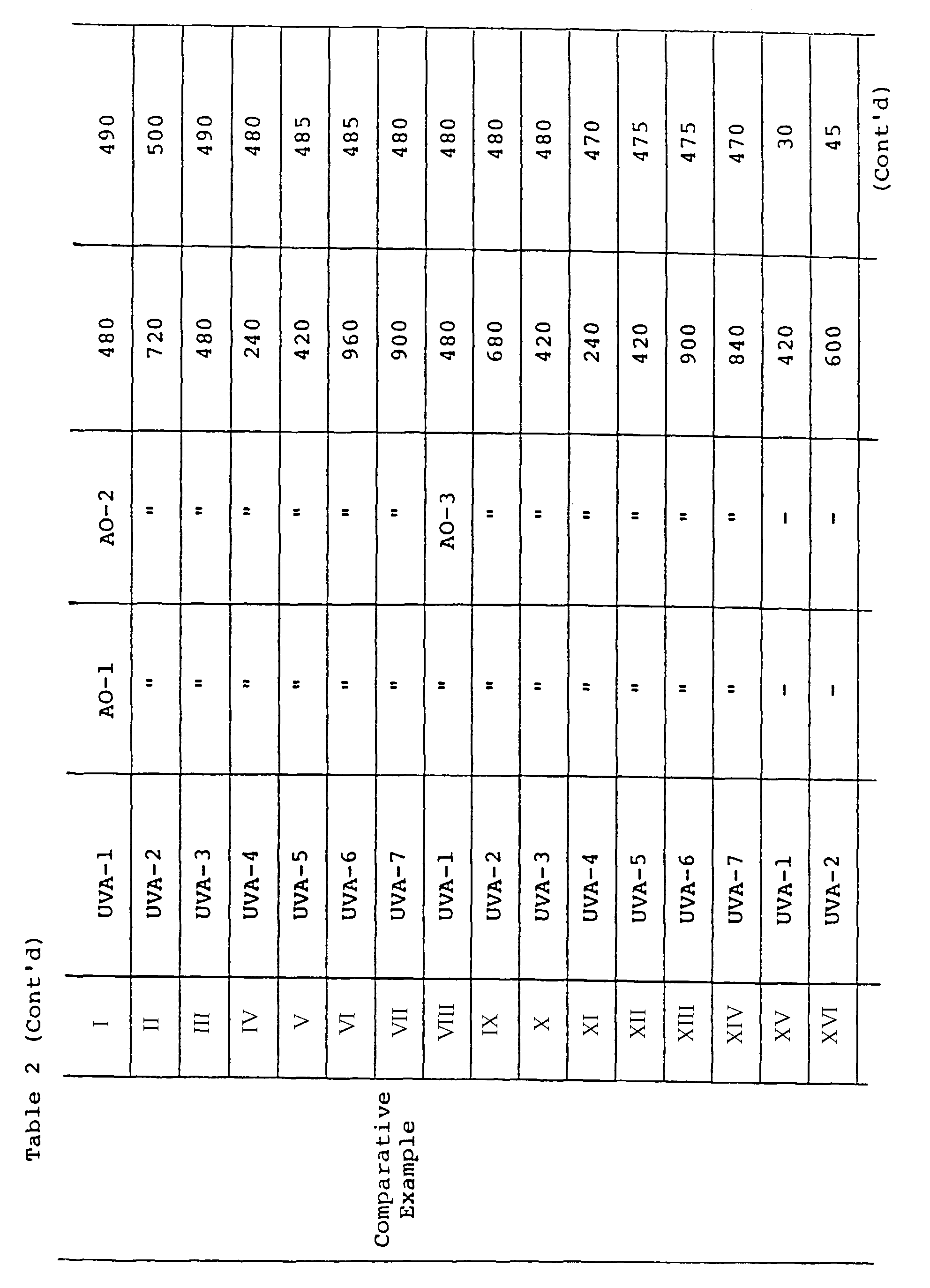

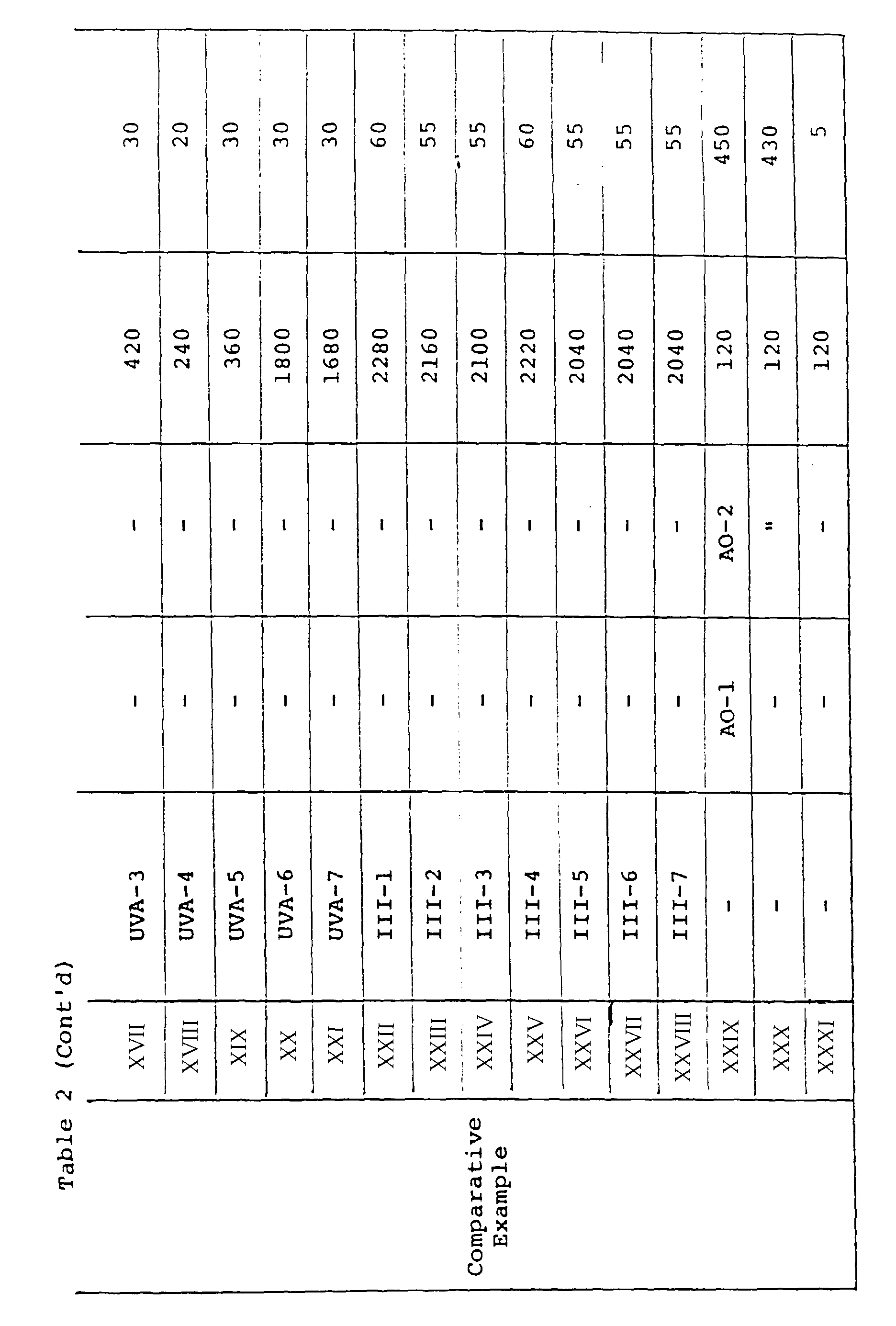

### Examples 14 to 20 and Comparative Examples XXXII to XXXV

Test compounds indicated in Table 3 were added to a 25% polyurethane dope (consisting of 25 parts by weight of polyurethane resin, 3.75 parts by weight of dimethylformamide and 71.25 parts by weight of tetrahydrofuran) in an amount of 1% by weight based on the polyurethane resin. The resulting mixture was cast on polyester film to 1.2 mm thickness and dried in a drier at 45°C for 1 hour.

No. 3 dumbbell test pieces were stamped out from the sheet thus obtained. The test pieces were irradiated with light in a fade meter (light source: ultraviolet ray carbon arc, black panel temperature: 63 ± 3°C) for 60 and 120 hours and then subjected to a tensile test (stretching velocity: 200 mm/min, temperature: 25°C) to determine percentage of retention of breaking strength.

The results obtained are shown in Table 3.

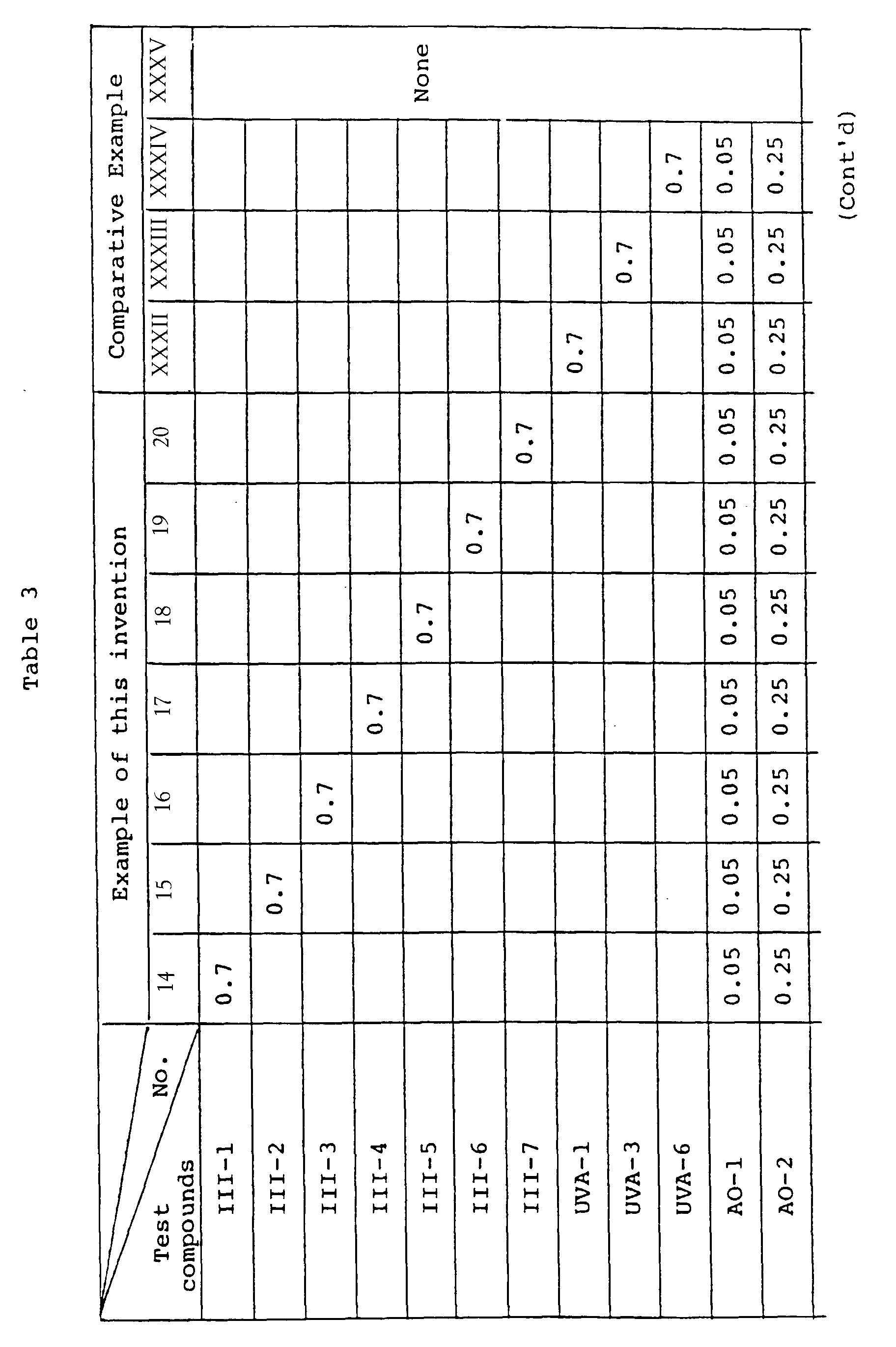

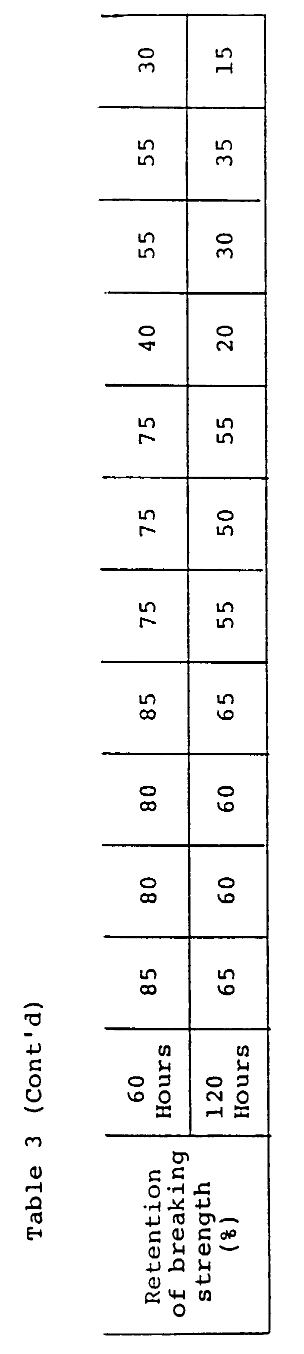

### Examples 21 to 27 and Comparative Examples XXXVI to XXXIX

A compound shown below was extrusion-molded at 200°C into pellets. The pellets were injection-molded at 230°C to form test pieces of 2 mm thickness.

The test pieces were irradiated in a fade meter (light source: ultraviolet ray carbon arc, black panel temperature: : 63 ± 3°C) for 1500 hours. The degree of discoloration was evaluated by the color difference ΔY_{I} from that of the specimen before irradiation.

The results obtained are shown in Table 4.

| Compounding composition | |
|---|---|
| ABS resin | 100 parts by wt. |
| Pentaerythritol tetrakis(3-(3,5-di-t-butyl-4-hydroxyphenyl)propionate) | 0.05 " |
| Distearyl 3,3'-thiodipropionate | 0.2 " |
| Test compound | 0.2 " |

**Table 4**

| | No. | Test compound | ΔY_{I} |
|---|---|---|---|
| Example of this invention | 21 | III-1 | 11.4 |
| | 22 | III-2 | 12.7 |
| | 23 | III-3 | 13.0 |
| | 24 | III-4 | 12.3 |
| | 25 | III-5 | 13.1 |
| | 26 | III-6 | 13.4 |
| | 27 | III-7 | 13.3 |
| Comparative Example | XXXVI | UVA-1 | 31.2 |
| | XXXVII | UVA-3 | 28.5 |
| | XXXVIII | UVA-6 | 28.1 |
| | XXXIX | None | 43.5 |

## Claims

1. A stabilized resin composition comprising a resin; a piperidine derivative represented by the general formula (III) 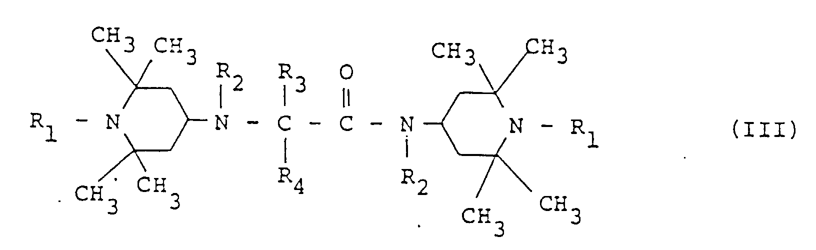 wherein R₁ is a hydrogen atom or an alkyl group having 1 to 3 carbon atoms, R₂ is a hydrogen atom or an alkyl group having 1 to 12 carbon atoms, and R₃ and R₄ are each independently an alkyl group having 1 to 12 carbon atoms; a phenol-type antioxidant;and a sulfur-containing antioxidant selected from the group consisting of dilauryl thiodipropionate, dimyristyl thiodipropionate, distearyl thiodipropionate, pentaerythritol tetrakis(3-dodecylthiopropionate) and 3,9-bis(2-dodecylthioethyl)-2,4,8,10-tetraoxaspiro[5.5]undecane.

2. A stabilized resin composition according to Claim 1 , wherein in the general formula (III), R₁ is a hydrogen atom or a methyl group, R₂ is a hydrogen atom or an alkyl group having 1 to 4 carbon atoms, and R₃ and R₄ are each independently an alkyl group having 1 to 7 carbon atoms.

3. A stabilized resin composition according to Claim 1 or 2, wherein the phenol-type antioxidant is one member selected from the group consisting of 2,6-di-t-butyl-4-methylphenol, n-octadecyl 3-(3,5-di-t-butyl-4-hydroxyphenyl)propionate, 2,2'-methylenebis(4-methyl-6-t-butylphenol), 4,4'-butylidenebis(3-methyl-6-t-butylphenol), 4,4'-thiobis(3-methyl-6-t-butylphenol), 2-t-butyl-6-(3-t-butyl-5-methyl-2-hydroxybenzyl)-4-methylphenyl acrylate, 1,1,3-tris(2-methyl-4-hydroxy-5-t-butylphenyl)butane, 1,3,5-trimethyl-2,4,6-tris(3-alkyl-5-t-butyl-4-hydroxybenzyl)benzene, 1,3,5-tris(3-alkyl-5-t-butyl-4-hydroxybenzyl) isocyanurate, 1,3,5-tris (3-(3-alkyl-5-t-butyl-4-hydroxyphenyl)propionyloxyethyl) isocyanurate, ethylene glycol bis(3,3-bis(4-hydroxy-3-t-butylphenly)butanoate, and pentaerythritol tetrakis(3-(3-alkyl-5-t-butyl-4-hydroxyphenyl)propionate).

4. A stabilized resin composition according to Claim 1, 2 or 3, wherein said resin is poly-α-olefins, α-olefin copolymers, copolymers of poly-α-olefin with a vinyl monomer, chlorinated polyethylene, EVA resin, polyvinyl chloride, methacrylic resin, polystyrene, high impact polystyrene, ABS resin, AES resin, MBS resin, polyethylene terephthalate, polybutylene terephthalate, polyamide, polyimide, polycarbonate, polyacetal, polyurethane, unsaturated polyester resin, or blended products of these resins with isoprene rubber, butadiene rubber, acrylonitrile-butadiene copolymer rubber, styrene-butadiene copolymer rubber or ethylene propylene rubber.

5. A stabilized resin composition according to any preceding claim, wherein the total amount of the piperidine derivative represented by the general formula (III), the phenol-type antioxidant and the sulfur-containing antioxidant is 0.01 to 5 parts by weight based on 100 parts by weight of the resin and the ratio of said piperidine derivative to the phenol-type antioxidant and the sulfur-containing antioxidant in this order is 1-20 : 1 : 1-15 by weight.

6. A stabilized resin composition according to any preceding claim, wherein a phosphite-type antioxidant is incorporated.

7. A stabilized resin composition according to any preceding claim, wherein a light stabilizer other than the piperidine derivative represented by the general formula (III) is incorporated.

## Patentansprüche

1. Stabilisierte Harzmasse, umfassend ein Harz, ein Piperidinderivat der allgemeinen Formel (III) 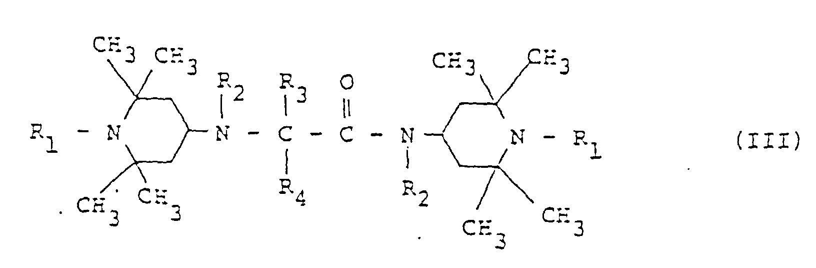 worin bedeuten:
R₁ ein Wasserstoffatom oder eine Alkylgruppe mit 1 bis 3 Kohlenstoffatom(en),
R₂ ein Wasserstoffatom oder eine Alkylgruppe mit 1 bis 12 Kohlenstoffatom(en) und
R₃ und R₄ unabhängig voneinander jeweils eine Alkylgruppe mit 1 bis 12 Kohlenstoffatom(en),
ein phenolartiges Antioxidationsmittel und ein schwefelhaltiges Antioxidationsmittel, ausgewählt aus der Gruppe Dilaurylthiodipropionat, Dimyristylthiodipropionat, Distearylthiodipropionat, Pentaerythrittetrakis(3-dodecylthiopropionat) und 3,9-Bis(2-dodecylthioethyl)-2,4,8,10-tetraoxaspiro[5.5]undecan.

2. Stabilisierte Harzmasse nach Anspruch 1, wobei in der allgemeinen Formel (III) bedeuten:
R₁ ein Wasserstoffatom oder eine Methylgruppe;
R₂ ein Wasserstoffatom oder eine Alkylgruppe mit 1 bis 4 Kohlenstoffatom(en) und
R₃ und R₄ jeweils unabhängig voneinander eine Alkylgruppe mit 1 bis 7 Kohlenstoffatom(en).

3. Stabilisierte Harzmasse nach Anspruch 1 oder 2, wobei es sich bei dem phenolartigen Antioxidationsmittel um ein solches aus der Gruppe 2,6-Di-tert.-butyl-4-methylphenol, n-Octadecyl-3-(3,5-di-tert.-butyl-4-hydroxyphenyl)propionat, 2,2'-Methylenbis-(4-methyl-6-tert.-butylphenol), 4,4'-Butylidenbis(3-methyl-6-tert.-butylphenol), 4,4'-Thiobis(3-methyl-6-tert.-butylphenol), 2-tert.-Butyl-6-(3-tert.-butyl-5-methyl-2-hydroxybenzyl)-4-methylphenylacrylat, 1,1,3-Tris(2-methyl-4hydroxy-5-tert.-butylphenyl)butan, 1,3,5-Trimethyl2,4,6-tris(3-alkyl-5-tert.-butyl-4-hydroxybenzyl) benzol, 1,3,5-Tris(3-alkyl-5-tert.-butyl-4-hydroxybenzyl)isocyanurat, 1,3,5-Tris(3-(3-alkyl-5-tert.-butyl-4-hydroxyphenyl)propionyloxyethyl)isocyanurat, Ethylenglykolbis(3,3-bis(4-hydroxy-3-tert.-butylphenyl)butanoat und Pentaerythrittetrakis(3 -(3-alkyl-5-tert.-butyl-4-hydroxyphenyl)propionat.

4. Stabilisierte Harzmasse nach Anspruch 1, 2 oder 3, wobei das Harz aus Poly-α-olefinen, α-Olefin-Copolymeren, Copolymeren von Poly-α-olefin mit einem Vinylmonomer, chloriertem Polyethylen, EVA-Harz, Polyvinylchlorid, Methacrylharz, Polystyrol, Polystyrol hoher Schlagzähigkeit, ABS-Harz, AES-Harz, MBS-Harz, Polyethylenterephthalat, Polybutylenterephthalat, Polyamid, Polyimid, Polycarbonat, Polyacetal, Polyurethan, ungesättigtem Polyesterharz oder Mischungsprodukten dieser Harze mit Isoprenkautschuk, Butadienkautschuk, Acrylnitril-Butadien-Copolymerkautschuk, Styrol/Butadien-Copolymerkautschuk oder Ethylen/Propylen-Kautschuk besteht.

5. Stabilisierte Harzmasse nach einem der vorhergehenden Ansprüche, wobei die Gesamtmenge an Piperidinderivat der allgemeinen Formel (III), phenolartigem Antioxidationsmittel und schwefelhaltigem Antioxidationsmittel 0,01 bis 5 Gew.-Teil(e), bezogen auf 100 Gew.-Teile des Harzes, und das Gewichtsverhältnis Piperidinderivat/phenolartiges Antioxidationsmittel/schwefelhaltiges Antioxidationsmittel (in der angegebenen Reihenfolge) 1-20/1/1-15 betragen.

6. Stabilisierte Harzmasse nach einem der vorhergehenden Ansprüche, in welche ein phosphitartiges Antioxidationsmittel eingearbeitet ist.

7. Stabilisierte Harzmasse nach einem der vorhergehenden Ansprüche, in die ein von dem Piperidinderivat der allgemeinen Formel (III) verschiedener Lichtstabilisator eingearbeitet ist.

## Revendications

1. Composition de résine stabilisée, comprenant une résine; un dérivé de pipéridine représenté par la formule générale (III) 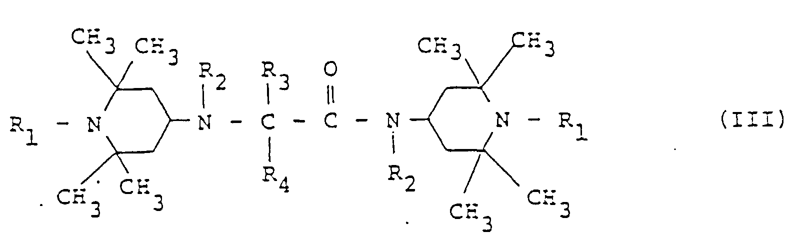 dans laquelle R₁ est un atome d'hydrogène ou un groupement alkyle à 1-3 atomes de carbone, R₂ est un atome d'hydrogène ou un groupement alkyle à 1-12 atomes de carbone, et R₃ et R₄ sont chacun, indépendamment l'un de l'autre, un groupement alkyle à 1-12 atomes de carbone; un antioxydant de type phénol; et un antioxydant contenant du soufre, choisi dans le groupe constitué par le thiodipropionate de dilauryle, le thiodipropionate de dimyristile, le thiodipropionate de distéaryle, le tétrakis(3-dodécylthiopropionate) de pentaérythritol et le 3,9-bis(2-dodécylthioéthyl)-2,4,8,10-tétraoxaspiro[5.5]undécane.

2. Composition de résine stabilisée selon la revendication 1 dans laquelle, dans la formule générale (III), R₁ est un atome d'hydrogène ou un groupement méthyle, R₂ est un atome d'hydrogène ou un groupement alkyle à 1-4 atomes de carbone, et R₃ et R₄ sont chacun, indépendamment l'un de l'autre, un groupement alkyle à 1-7 atomes de carbone.

3. Composition de résine stabilisée selon la revendication 1 ou 2, dans laquelle l'antioxydant de type phénol est un membre choisi dans le groupe constitué par le 2,6-di-t-butyl-4-méthylphénol, le 3-(3,5-di-t-butyl-4-hydroxyphényl)propionate de n-octadécyle, le 2,2'-méthylènebis(4-méthyl-6-t-butylphénol), le 4,4'-butylidènebis(3-méthyl-6-t-butylphénol), le 4,4'-butylidènebis(3-méthyl-6-t-butylphénol), le 4,4'-thiobis(3-méthyl-6-t-butylphénol), l'acrylate de 2-t-butyl-6-(3-t-butyl-5-méthyl-2-hydroxybenzyl)-4-méthylphényle, le 1,1,3-tris(2-méthyl-4-hydroxy-5-t-butylphényl)butane, le 1,3,5-triméthyl-2,4,6-tris(3-alkyl-5-t-butyl-4-hydroxybenzyl)benzène, l'isocyanurate de 1,3,5-tris(3-alkyl-5-t-butyl-4-hydroxybenzyle), l'isocyanurate de 1,3,6-tris[3-(3-alkyl-5-t-butyl-4-hydroxyphényl)propionyloxyéthyle], le bis[3,3-bis(4-hydroxy-3-t-butylphényl)butanoate] d'éthyléneglycol et le tétrakis[3-(3-alkyl-5-t-butyl-4-hydroxyphényl)propionate] de pentaérythritol.

4. Composition de résine stabilisée selon l'une quelconque des revendications 1 à 3, dans laquelle ladite résine est choisie parmi les poly-α-oléfines, les copolymères d'α-oléfine, les copolymères de poly-α-oléfine avec un monomère vinylique, le polyéthylène chloré, la résine EVA, le poly(chlorure de vinyle), une résine méthacrylique, un polystyrène, un polystyrène haute résilience, une résine ABS, une résine AES, une résine MBS, un poly(téréphtalate d'éthylène), un poly(téréphtalate de butylène), un polyamide, un polyimide, un polycarbonate, un polyacétal, un polyuréthanne, une résine de polyester insaturé ou des produits de mélange de ces résines avec du caoutchouc isoprène, du caoutchouc butadiène, du caoutchouc copolymère acrylonitrile/butadiène, du caoutchouc copolymer styrène/butadiène ou du caoutchouc éthylène/propylène,

5. Composition de résine stabilisée selon l'une quelconque des revendications précédentes, dans laquelle la quantité totale du dérivé de pipéridine représente par la formule générale (III), de l'antioxydant de type phénol et de l'antioxydant contenant du soufre est de 0,01 à 5 parties en poids sur la base de 100 parties en poids de la résine, et le rapport dudit dérivé de pipéridine à l'antioxydant de type phénol et à l'antioxydant contenant du soufre est, dans cet ordre, de 1-20 : 1 : 1-15 en poids.

6. Composition de résine stabilisée selon l'une quelconque des revendications précédentes, dans laquelle un antioxydant de type phosphite est incorporé.

7. Composition de résine stabilisée selon l'une quelconque des revendications précédentes, dans laquelle un stabilisant à la lumière autre que le dérivé de pipéridine représenté par la formule générale (III) est incorporé.
